# EUROPEAN PATENT APPLICATION

(11) **EP 4 390 955 A1**
(43) Date of publication of application: **26.06.2024**
(21) Application number: 23212554.2
(22) Date of filing: 28.11.2023
(51) Int. Cl.: G16H 20/30

(54) **AUTOMATED MONITORING OF EXERCISE DEVICES**

(30) Priority: 02.12.2022 SE 2251408
(71) Applicant: Lumos Holdings US Acquisition Co., Rosemont, IL 60018 (US)
(72) Inventor: BENGTSSON, Henrik, Rosemont, 60018 (US); RYDBERG, Jens, Rosemont, 60018 (US); HALLQVIST, Magnus, Rosemont, 60018 (US); FREDLUND, Anders, Rosemont, 60018 (US); BOROWSKI, Jimmy, Rosemont, 60018 (US)
(74) Representative: Neij & Lindberg AB

(57) **Abstract**

A monitoring arrangement for use with an exercise device. The monitoring arrangement includes an inertial measurement device that detects an exercise activity of the exercise device and an activity measurement device having an active mode and a disabled mode. The activity measurement device is operable to quantify the exercise activity of the exercise device when in the active mode, where the activity measurement device uses less power when in the disabled mode than in the active mode. A control unit is configured to detect, via the inertial measurement device, a start of the exercise activity, to cause the activity measurement device to operate in the active mode upon detecting the start of the exercise activity, and to operate the activity measurement device to generate measurement data that represents the exercise activity when in the active mode. The activity measurement device is defaulted to the disabled mode to save power.

## Description

### CROSS REFERENCE TO RELATED APPLICATION

This application claims the benefit of Swedish Patent Application No. 2251408-7, filed December 2, 2022, which is incorporated herein by reference in its entirety.

### FIELD

The present disclosure relates generally to exercise devices, and, more particularly, to techniques for monitoring exercise activities of exercise devices.

### BACKGROUND

Exercise and physical fitness are steadily gaining in popularity. The growing interest in physical fitness is reflected by the growing number of gyms found in both public and private settings.

Exercise devices are often used for physical exercise, for example exercise machines in which stacked weight plates are lifted by the user against the action of gravity. Conventionally, the user has to keep a manual record of the exercises performed on the machines and the outcome of the respective exercise.

Recently, automated systems have been developed to help a user to track and record progress on exercise machines. Such automated systems comprise a local detector on the respective exercise machine. The local detector is configured to determine exercise data that represents an exercise activity performed by a user of the exercise machine and transfer the exercise data to a local or remote device for storage and/or presentation. An example of such a system is described in WO2019/147174, which is incorporated by reference herein in its entirety. In this prior art system, the local detector is configured to broadcast exercise data for receipt by an observer unit, which is installed in the facility of the exercise machine and is configured to forward the exercise data to a server, which in turn forwards the exercise data to a user device carried by the user, thereby providing real-time feedback to the user. In addition, since the exercise data from each exercise machine is routed via the server, it is possible for the server to collect usage data for a large number of exercise machines, for example all machines in a gym. In a variation, the observer unit is instead installed in the user device. In this variation, usage data for plural exercise machines cannot be collected in a centralized manner.

Power consumption is a concern in automated systems for monitoring of exercise devices. The local detector is typically battery-powered. In a facility with many exercise machines, for example a gym, replacement of batteries may be time consuming, especially since the local detectors on different exercise machines may run low on battery at different times. There is thus a general desire to reduce the power consumption of such automated monitoring systems.

### SUMMARY

This Summary is provided to introduce a selection of concepts that are further described below in the Detailed Description. This Summary is not intended to identify key or essential features of the claimed subject matter, nor is it intended to be used as an aid in limiting the scope of the claimed subject matter.

Objectives of the present disclosure include at least partly overcome one or more limitations of the prior art. Another objective is to reduce the power consumption of a monitoring arrangement for use with an exercise device. A further objective is to enable centralized collection of usage data for a plurality of exercise devices. Yet another objective is to enable the centralized collection of usage data also when the user does not have a user device that is configured or activated to provide exercise feedback.

One or more of these objectives, as well as further objectives that may appear from the description below, are at least partly achieved by a monitoring arrangement according to the independent claims, embodiments thereof being defined by the dependent claims.

According to various aspects of the present disclosure, the monitoring arrangement comprises a communication device, an inertial measurement device and an activity measurement device. The activity measurement device is in a disabled mode by default, whereas the inertial measurement device is active and used for detecting start of the exercise activity. To reduce power consumption, the monitoring arrangement is configured to selectively switch the activity measurement device into an active mode to generate measurement data that quantifies the exercise activity, and specifically when there is a mobile device present at the exercise device at the start of the exercise activity. The provision of the measurement data results in first exercise data, which is transmitted from the monitoring arrangement to the mobile device, thereby enabling the first exercise data to be presented to user, for example as real-time feedback about the progress of the exercise activity. If the monitoring arrangement is instead unable to detect presence of a mobile device when the exercise activity is started, the monitoring arrangement calculates second exercise data by use of a measurement signal from the inertial measurement device. Further, if the mobile device is deemed to be absent, the activity measurement device is either maintained in its default mode, i.e. disabled, or set in an intermediate low-power mode. This results in a significantly reduced power consumption of the monitoring arrangement, since the activity measurement device is only operated in the active mode when a mobile device that is capable of making use of the first exercise data is actually present at the exercise device when the exercise activity is started. The second exercise data represents the exercise activity and is not transmitted to a mobile device, which is absent. Instead, the second exercise data may be stored in the monitoring arrangement or transmitted for receipt by one or more receiving devices that are installed in the facility that houses the exercise device. Thereby, the second exercise data is available for quantifying the usage of the exercise machine. If monitoring arrangements are installed on a plurality of exercise devices, the second exercise data may be collected, via the receiving device(s), for centralized analysis of the usage of the exercise devices, for example to identify a need for maintenance.

Thus, in the monitoring arrangement, clever use is made of the already-present inertial measurement device to quantify the usage of the exercise machine whenever the exercise device is operated by users that do not have a mobile device that is configured for use with the monitoring arrangement. The additional use of the inertial measurement device does not significantly increase power consumption, given that inertial measurement devices are generally power efficient and typically active at all times anyway to enable the monitoring arrangement to detect the start of an exercise activity.

Another aspect of the present disclosure generally relates to a monitoring arrangement for use with an exercise device. The monitoring arrangement includes an inertial measurement device configured to detect an exercise activity of the exercise device, and an activity measurement device separate from the inertial measurement device. The activity measurement device has an active mode and a disabled mode. The activity measurement device is operable to quantify the exercise activity of the exercise device when in the active mode. The activity measurement device uses less power when in the disabled mode than in the active mode. A control unit is configured to detect, via the inertial measurement device, a start of the exercise activity, to cause the activity measurement device to operate in the active mode upon detecting the start of the exercise activity, and to operate the activity measurement device to generate measurement data that represents the exercise activity when in the active mode. The activity measurement device is defaulted to the disabled mode to save power.

In certain examples, the monitoring arrangement further includes a communication device, where the control unit is further configured to transmit first exercise data that represents the measurement data from the activity measurement device via the communication device. In further examples, the communication device is configured to communicate wirelessly. In further examples, the control unit is further configured to determine a presence or absence of a mobile device and, when the mobile device is present, to transmit the first exercise data to the mobile device. In further examples, the control unit is further configured to determine a presence or absence of a mobile device and, when the mobile device is absent, to calculate second exercise data that represents the exercise activity using a measurement signal from the inertial measurement device, the second exercise data being different than the first exercise data. In further examples, the first exercise data is real-time data and the second exercise data is non-real-time data. In further examples, the control unit is further configured to transmit the second exercise data to a receiver device via the communication device, the receiver device being different from the mobile device. In further examples, the control unit is further configured to detect a termination of the exercise activity and to transmit the second exercise data when the termination of the exercise activity is detected. In further examples, the termination is detected using the measurement signal from the inertial measurement device. In further examples, the control unit causes the activity measurement device to operate in an intermediate mode when the mobile device is absent, the activity measurement device using less power when in the intermediate mode than in the active mode and more power when in the intermediate mode than in the disabled mode. In further examples, the control unit is further configured to detect a termination of the exercise activity based at least in part on the measurement data from the activity measurement device when operated in the intermediate mode, and to transmit the second exercise data when the termination of the exercise activity is detected. In further examples, the control unit is further configured to calculate second exercise data that represents the exercise activity also using the measurement data from the activity measurement device when operated in the intermediate mode. In further examples, the second exercise data is indicative of a count of exercise movements performed during the exercise activity. In further examples, the first exercise data that represents the measurement data from the activity measurement device is transmitted in time synchronization with the exercise activity.

In another example, the activity measurement device is operable to determine a momentary position of one of the exercise device and a weight of the exercise device.

In another example, the activity measurement device has a communication device and the control unit is further configured to transmit first exercise data that represents the measurement data from the activity measurement device via the communication device, where the first exercise data represents a movement trajectory of the one of the exercise device and the weight of the exercise device. In further examples, the first exercise data includes at least one of: an indication of a start of an exercise movement performed as part of the exercise activity, an indication of a completion of the exercise movement performed as part of the exercise activity, a count of repetitive exercise movements performed during the exercise activity, and an indication of a load being lifted during an exercise movement.

Another aspect of the present disclosure generally relate to a method for monitoring exercise activity for an exercise device. The method includes detecting the exercise activity via an inertial measurement device, and detecting, via a control unit, a start of the exercise activity from the inertial measurement device. The method further includes causing an activity measurement device to operate in an active mode when the start of exercise activity is detected, where the activity measurement device being defaulted to a disabled mode that uses less power than the active mode when the start of exercise activity is undetected. The method further includes generating, via the activity measurement device when in the active mode, measurement data that quantifies the exercise activity, and communicating a first exercise data that represents the measurement data from the activity measurement device when the activity measurement device is in the active mode.

In certain examples, the method further includes determining whether a mobile device separate from the exercise device is present or absent and, when the mobile device is absent, calculating and communicating a second exercise data that represents the exercise activity using a measurement signal from the inertial measurement device, the second exercise data being different than the first exercise data. In further examples, the first exercise data is real-time data and the second exercise data is non-real-time data, and the second exercise data is communicated to a receiver device that is different from the mobile device.

It should be recognized that the different aspects described throughout this disclosure may be combined in different manners, including those than expressly disclosed in the provided examples, while still constituting an invention accord to the present disclosure.

Still other objectives, aspects and technical advantages, as well as further features and embodiments will appear from the following detailed description, from the attached claims as well as from the schematic drawings.

### BRIEF DESCRIPTION OF DRAWINGS

FIG. 1 depicts an example exercise device provided with an arrangement for monitoring exercise activity.
FIG. 2 is a block diagram of an example monitoring arrangement.
FIGS 3A-3B depict an example monitoring arrangement in relation to an exercise machine in a rest state and a loaded state, respectively.
FIG. 4 depicts an example monitoring arrangement in relation to a handheld exercise device.
FIG. 5 illustrates an example data management system.
FIG. 6 is a block diagram of an example mobile device in the data management system of FIG. 5.
FIGS 7A-7B are flow charts of methods of operating a monitoring arrangement in accordance with embodiments.
FIGS 8A-8B are sequence diagrams of example operation of the data management system in FIG. 5 for two different use cases.

### DETAILED DESCRIPTION

Embodiments will now be described more fully hereinafter with reference to the accompanying drawings, in which some, but not all, embodiments are shown. Indeed, the subject of the present disclosure may be embodied in many different forms and should not be construed as limited to the embodiments set forth herein; rather, these embodiments are provided so that this disclosure may satisfy applicable legal requirements.

Also, it will be understood that, where possible, any of the advantages, features, functions, devices, and/or operational aspects of any of the embodiments described and/or contemplated herein may be included in any of the other embodiments described and/or contemplated herein, and/or vice versa. In addition, where possible, any terms expressed in the singular form herein are meant to also include the plural form and/or vice versa, unless explicitly stated otherwise. As used herein, "at least one" shall mean "one or more" and these phrases are intended to be interchangeable. Accordingly, the terms "a" and/or "an" shall mean "at least one" or "one or more", even though the phrase "one or more" or "at least one" is also used herein. As used herein, except where the context requires otherwise owing to express language or necessary implication, the word "comprise" or variations such as "comprises" or "comprising" is used in an inclusive sense, that is, to specify the presence of the stated features but not to preclude the presence or addition of further features in various embodiments.

As used herein, the terms "multiple", "plural" and "plurality" are intended to imply provision of two or more elements. The term "and/or" includes any and all combinations of one or more of the associated listed elements.

It will furthermore be understood that, although the terms first, second, etc. may be used herein to describe various elements, these elements should not be limited by these terms. These terms are only used to distinguish one element from another. For example, a first element could be termed a second element, and, similarly, a second element could be termed a first element, without departing the scope of the present disclosure.

Well-known functions or constructions may not be described in detail for brevity and/or clarity. Unless otherwise defined, all terms (including technical and scientific terms) used herein have the same meaning as commonly understood by one of ordinary skill in the art to which this disclosure belongs.

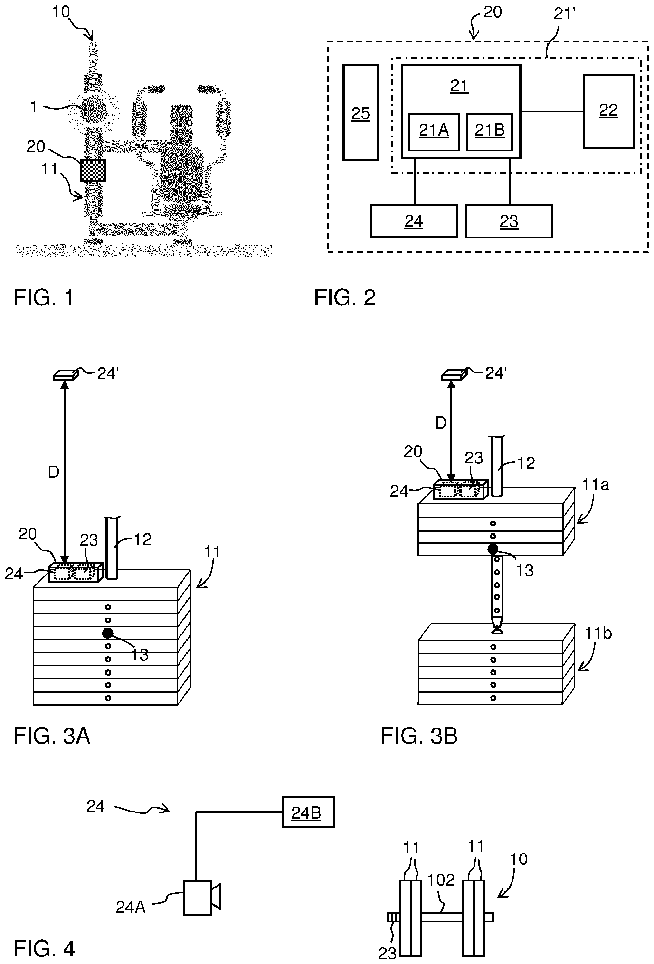

FIG. 1 is an isometric view of a stacked weight exercise machine 10 having a plurality of weights 11. The exercise machine 10 is equipped with a monitoring arrangement 20, abbreviated MA, which enables the exercise machine 10 to be included in an automated system for tracking exercise activity and providing feedback to users of the exercise machine 10. Even if the MA 20 is connected to mains power, it may be desirable to reduce power consumption.

As noted in the Background section, power consumption is a concern in such automated systems, for example if the MA 20 is self-powered by batteries or another on-board power source. In aforesaid WO2019/147174, the MA (local detector) comprises a repetition detector, which is arranged on a moveable weight in the exercise machine and is configured to detect repetitions by use of a range finder. To reduce power consumption, the range finder is in sleep mode by default and is selectively activated by an accelerometer detecting movement of the repetition detector and thus of the moveable weight. One potential drawback is that the range finder may be erroneously activated by unrelated vibrations, for example caused by exercise activities performed on neighboring exercise machines, people jumping, loud music, etc. Frequent and unwarranted activations of the range finder will result in an increased power consumption and accelerated draining of battery power.

Embodiments described herein aim at limiting the power consumption of the MA. Embodiments also aim at devising an MA, which is capable of providing feedback about an exercise activity to the user, preferably in real-time, through a mobile device, and which is also capable of providing usage data about the use of an exercise device to a centralized device.

The description uses the term "exercise device" to include both exercise machines and so-called free weights. An exercise machine is any type of stationary device that allows a user to perform an exercise activity that involves moving a moveable element of the exercise machine (cf. stacked weights 11 in FIG. 1). Free weights include dumbbells, barbells, medicine balls, kettlebells, etc. Any type of exercise device may be equipped with or associated with an MA20.

The term "exercise activity" refers to any use of an exercise device that requires physical effort of a user and is carried out to sustain or improve the user's health and/or fitness. Often, an exercise activity involves a user performing a predefined exercise movement of the exercise device or a moveable element of the exercise device. Conventionally, performance of one predefined exercise movement is denoted a "repetition", and a sequence of repetitions is denoted a "set". Often, a repetition comprises an eccentric phase and a concentric phase, in any order. Typically, a set is performed without pause between the repetitions, whereas the user pauses between sets if more than one set is performed.

The term "real-time feedback" as used herein implies that feedback is given in time synchronization with an on-going exercise activity. In one example, presentation of real-time feedback involves incrementing a presented number of repetitions in time synchronization with the user's performance of predefined exercise movements. In another example, presentation of real-time feedback involves presenting a movement trajectory of the exercise device, or a moveable element of the exercise device, in time synchronization with the user's performance of predefined exercise movements.

FIG. 2 is block diagram of an example MA 20 in accordance with an embodiment. The MA 20 is configured to be mounted on or be otherwise associated with an exercise device. The components illustrated in FIG. 2 may be located in a housing or be distributed. A control unit 21 ("control circuitry") is configured to implement logic for controlling the MA 20. In the illustrated example, the control unit 21 comprises a combination of a processor 21A and a memory 21B. The memory 21B may store program instructions for execution by the processor 21A to implement the operation of the control unit 21. The program instructions may be supplied to the processor 21A on a computer-readable medium, which may be a tangible (non-transitory) product (for example, magnetic medium, optical disk, read-only memory, flash memory, etc.) or a propagating signal. The processor 21A may be a generic processor, for example a microprocessor, microcontroller, CPU, DSP (digital signal processor), GPU (graphics processing unit), etc., or a specialized processor, such as an ASIC (application specific integrated circuit) or an FPGA (field programmable gate array), or any combination thereof. The memory 21B may include volatile and/or non-volatile memory such as read only memory (ROM), random access memory (RAM) or flash memory.

The control unit 21 is connected to a communication device 22 and is operable to cause the communication device 22 to transmit a wireless signal. For brevity, the communication device 22 is denoted COMM in the following. In some embodiments, COMM 22 is configured for wireless short-range communication. In one implementation example, COMM 22 is configured for Bluetooth communication and comprises at least one Bluetooth transmitter. As used herein, a "Bluetooth transmitter" or "BT transmitter" refers to any wireless radio device capable of transmitting data in compliance with a Bluetooth SIG standard in any version. The term "transmitter" also encompasses transceivers. One specific type of BT transmitter is a BLE transmitter, which is configured to operate according to the Bluetooth Low Energy standard. Bluetooth Low Energy is also known as Bluetooth LE and Bluetooth Smart. However, COMM 22 is not restricted to communication by Bluetooth but may use any wireless communication technique, including but not limited to Bluetooth, Wi-Fi, ANT, Zigbee, Z-wave, UWB, LR-WPAN, LoRa, ambient backscatter communication (ABC), etc., either singly or in any combination.

In some embodiments, COMM 22 is operable in a first mode, in which it broadcasts data packages, and a second mode, in which it establishes an end-to-end connection and transmits a stream of data on the end-to-end connection.

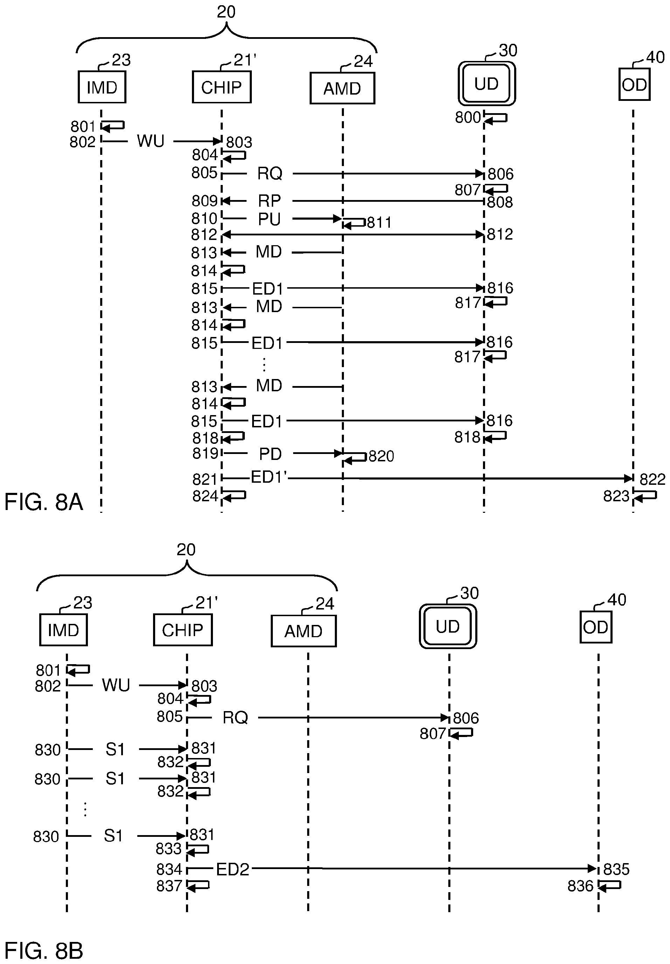

In some embodiments, the control unit 21 and COMM 22 are located in a single package 21', for example an integrated circuit or chip. Embodiments involving such a single package 21' are described below with reference to FIGS 8A-8B.

The MA 20 further comprises an inertial measurement device 23, abbreviated IMD. The IMD 23 is a power-efficient device which is responsive to inertial changes that occur when the exercise device 10 is deployed in an exercise activity. The IMD 23 comprises one or more sensors for measuring one or more inertial parameters, including but not limited to one or more of acceleration or orientation. For example, the sensor(s) may include one or more accelerometers, one or more gyrometers, or one or more magnetometers, or any combination thereof. In one embodiment, the IMD 23 is a MEMS-based IMU. The IMD 23 may output a measurement signal that includes raw data generated by the included sensor(s), or refined data that is generated by the IMD 23 based on the raw data. For example, the refined data may be indicative of one or more of acceleration, velocity, angular rate, specific force, relative position, or orientation. The IMD 23 may be a commercially available device or a customized device. For example, there are commercially available IMUs, for use in wearable devices, with an operative current in the range of 30-300 µA or even less.

The MA 20 further comprises an activity measurement device 24, abbreviated AMD. The AMD 24 comprises one or more sensors and is operable to quantify an exercise activity that is performed by use of the exercise device 10. Examples of the AMD 24 are given below with reference to FIGS 3-4. The AMD 24 differs from the IMD 23 by being operable to provide measurement data of higher precision and/or relevance for evaluating the exercise activity. The AMD 24 has at least two operating modes; a disabled mode ("sleep mode"), in which the AMD 24 is disabled, and a high-power mode ("active mode"), in which the AMD 24 generates and outputs measurement data. The AMD 24 is in the sleep mode by default. In the active mode, the AMD 24 has a significantly higher power consumption than the IMD 23. In some embodiments, the power consumption of the AMD 24 in the active mode is in the range of at least 10-1,000 times the power consumption of the IMD 23. In some embodiments, the AMD 24 further has an intermediate mode, in which the AMD 24 also operates to generate measurement data but at a lower power consumption than in the active mode. For example, the AMD 24 may generate the measurement data at a lower rate and/or accuracy in the intermediate mode. In some embodiments, the power consumption is reduced by at least 50%, and preferably at least 75%, in the intermediate mode compared to the active mode.

The IMD 23 and the AMD 24, respectively, may be connected by wire or wirelessly to the control unit 21.

In the example of FIG. 2, the MA 20 also comprises a power source 25 for supplying power to the electrical components of the MA 20. The power source 25 may include one or more of a battery, a fuel cell, an energy harvesting unit (photovoltaic cell, vibration-powered generator, a thermoelectric generator, etc.), an electrical connector for mains power, etc.

FIGS 3A-3B are given to provide a non-limiting example of an exercise machine 10 and the installation of an MA 20 therein. In the illustrated example, the exercise machine 10 comprises a lifting mechanism 12 and an engaging member ("selector pin") 13 for selectively engaging a number of stacked weights to the lifting mechanism 12. The lifting mechanism 12 may be coupled, in a manner well known to the skilled person, to one or more gripping or pushing members via one or more cables, belts, rods, etc. In the illustrated example, the lifting mechanism 12 includes a support member having a rod-shaped portion, configured to pass vertically through corresponding holes in the weights 11. The support member 12 may further include a top portion, such as a fixed top weight.

In exercise machines comprising stacked weights, the user may typically select how many of the weights should be used or engaged in an exercise activity, by inserting the selector pin 13 in one of the weights. During an exercise activity, the user will then lift the selected weights, as exemplified in FIG. 3B, where the selected portion 11a of the stacked weights is lifted in relation to a remaining portion 11b. The exercise machine 10 thus has a "rest state" (FIG. 3A), which is attained when the user does not apply force to the machine 10. When not in the rest state, the machine 10 is in a "loaded state" (FIG. 3B). The exercise machine may or may not attain the rest state between repetitions, depending on the training schedule of the specific user.

In the example of FIGS 3A-3B, the MA 20 is a unitary device that is arranged on top of the stacked weights 11. The MA 20 is thus moving when a user performs an exercise activity in the machine 10. It may be noted that all parts of the MA 20 need not be arranged for movement with the stacked weights 11. If the MA 20 is a distributed arrangement, it is sufficient that the IMD 23 and the AMD 24 are arranged on the stacked weights 11.

In the illustrated example, the AMD 24 is or comprises a range sensor, which is configured to measure a parameter representative of a distance D to a stationary element 24'. For example, the element 24' may be attached to or part of the frame of the machine 10. The measured distance D is representative of a momentary distance between the top of the weights 11 and the reference element 24', and thus a momentary position of the weights that have been selected by the user. Thereby, the AMD 24, in the active mode, is operable to accurately track and quantify an exercise activity performed by user of the exercise machine 10. For example, the AMD 24 may be operable to provide a time sequence of momentary distances that represent a movement trajectory of the selected weights. The provision of such a time sequence makes it possible to provide real-time feedback to the user during the exercise activity. As noted above, the AMD 24 may also be operable in an intermediate mode with lower power consumption than the active mode. For example, the range sensor may be operated to generate measurement values (D) at a lower rate in the intermediate mode than in the active mode.

The range sensor may be a time-of-flight (ToF) sensor. Time-of-flight (ToF) is an established technique for distance determination and involves measuring the roundtrip time of a signal provided by a source in the AMD 24 onto the reference element 24', which is reflective to the signal from the AMD 24. The AMD 24 is configured to calculate the distance D as half the roundtrip time multiplied by the propagation speed of the signal. The signal may be an ultrasound signal or an electromagnetic signal. In some embodiments, the signal is a light signal in the ultraviolet, visible or infrared wavelength range. The light signal may be generated by a laser or an LED.

In a variant, the positions of the AMD 24 and the stationary element 24' in FIGS 3A-3B are reversed.

In a further variant, the AMD 24 is configured to also quantify how many weights that are lifted during the exercise activity. For example, the AMD 24 may be configured to measure the distance to the selector pin 13. This distance is indicative to the number of selected weights. For example, the AMD 24 may comprise another range sensor, which is configured to emit a signal for reflection on the selector pin 13.

The AMD 24 is not limited to range sensors. In other examples, the AMD 24 comprises a triangulation system, a trilateration system or a computer vision system for quantifying an exercise activity that is performed by use of an exercise device. All of these systems are capable of determining the momentary position of one or more weights in the exercise machine 10 or the momentary position of the exercise device 10 itself (cf. FIG. 4).

FIG. 4 is a side view of an exercise device 10 in the form of a dumbbell having weights 11 arranged on a gripping rod 102. In FIG. 4, the MA is represented by an IMD 23 and an AMD 24. The IMD 23 is attached to or integrated with the dumbbell 10 to be responsive to the movement of the dumbbell 10 during an exercise activity. The AMD 24 is a computer vision system that is installed at the location of the dumbbell 10. A digital camera 24A is arranged to capture video images of the dumbbell 10, and possibly the user, during an exercise activity, and a processing device 24B is configured to process the video images to quantify the exercise activity. For example, the processing device 24B may be configured to provide a time sequence of momentary positions that represent a movement trajectory of the dumbbell 10, and optionally the user or a body part thereof.

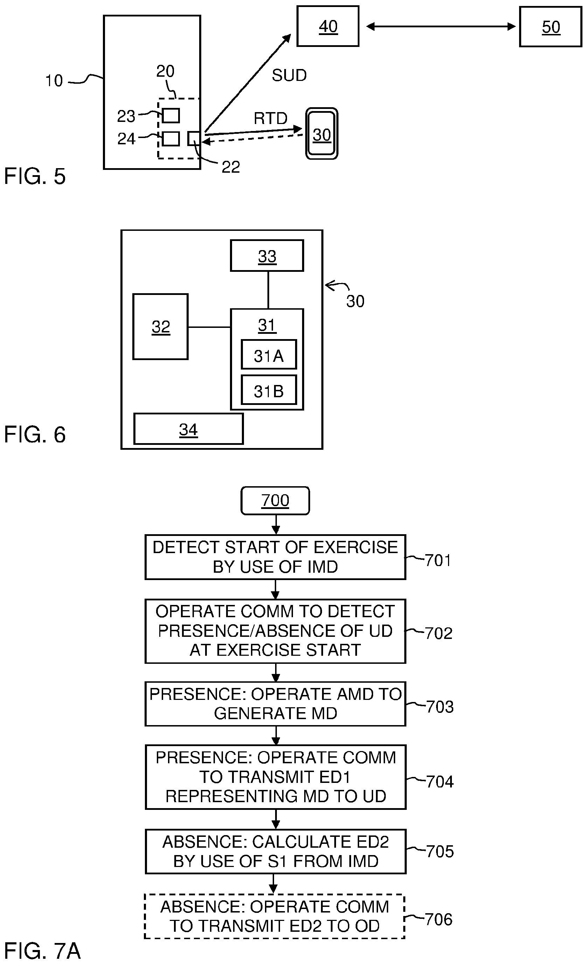

FIG. 5 illustrates a data management system in relation to a plurality of exercise devices 10 (one shown), which are provided with a respective MA 20. The plurality of exercise devices 10 are located in a common facility, for example a gym. The system further comprises mobile user devices 30 (one shown). Each mobile user device 30, also denoted user device or UD, may be any type of electronic device which is capable of being carried, held or worn by a user. Such electronic devices include handheld devices, such as mobile phones, smartphones, tablets, laptops, gaming consoles, etc., as well as wearable computers ("wearables"), such as smart glasses, smart watches, headsets, badges, bracelets, fitness trackers, etc. The system further comprises at least one observer device 40, abbreviated OD, which is configured to receive incoming data from the MAs 20 and relay the incoming data, or part thereof, to a back-end device 50, for example a server. The OD 40 may be connected to the back-end device 50 over a network, such as a WAN, LAN, PAN or any combination thereof.

As indicated by a solid arrow in FIG. 5, the respective MA 20 is operable to transfer, by use of COMM 22, real-time data, RTD, to the UD 30 of the current user of the exercise device 10. As indicated by a dashed arrow, the communication between the MA 20 and the UD 30 may, but need not, be bi-directional. As indicated by a further solid arrow in FIG. 5, the MA 20 is also operable to transfer summary usage data, SUD, by use of COMM 22, to the OD 40.

The RTD represents an on-going exercise activity and is transferred so as to allow the UD 30 to provide real-time feedback to the user of the exercise device 10. In some embodiments, the MA 20 is configured to broadcast a data packet containing RTD for receipt by the UD 30, and optionally by the OD 40. In some embodiments, the MA 20 is configured to establish an end-to-end connection with the UD 30 and transfer RTD over the end-to end connection.

The SUD summarizes the usage of the exercise device 10 during the exercise activity and may be transferred when the exercise activity is terminated. In some embodiments, SUD comprises a count of the total number of repetitions that have been performed by the user during the exercise activity, including all sets performed. The SUD is transferred to the back-end device 50 via the OD 40 so as to allow the back-end device 50 to evaluate the usage of the different exercise devices in the facility. For example, the back-end device 50 may store SUD in a database and/or evaluate SUD for detection or prediction of a need for maintenance of an exercise device.

In some embodiments, the MA 20 is configured to determine SUD and broadcast a data packet containing the SUD for receipt by the OD 40. Here, the OD 40 may comprise a communication interface for monitoring communication signals transmitted by the MAs 20 that are installed in the facility. Specifically, the OD 40 is operable to determine whether an incoming data package comprises SUD, and forward the SUD to the back-end device 50.

In some embodiments, the OD 40 is configured to, in addition to listening for data packets containing SUD, also listen for data packets containing RTD and generate SUD based on the RTD. Thus, OD 40 may be configured to process the RTD as received over time from an MA 20 to determine SUD, and then transfer the thus-determined SUD to the back-end device 50.

In the foregoing, it is implicit that the respective MA 20 stores an identifier (MAID) which is unique within the system and which is included in outgoing transmissions to identify the origin of the RTD and the SUD. In some embodiments, for example as indicated in FIG. 1, the respective exercise device 10 has an electronic tag 1, which may be attached to or arranged nearby the exercise device 10. The tag 1 enables a user to associate its UD 30 with the exercise device 10, by executing a "docking operation", before initiating an exercise activity on the exercise device 10. In the docking operation, the UD 30 is brought close to the tag 1, whereby a tag identifier is wirelessly transferred from the tag 1 to the UD 30, which is thereby capable of identifying the exercise device 10, for example by querying a server (not shown). For example, the server may return metadata describing the exercise device 10 to the UD 30. Thereby, the UD 30 is capable of presenting the RTD to the user together with the metadata. The server may also return the MAID of the MA 20. Thereby, by use of the MAID, the UD 30 is capable of detecting transmissions originating from a specific MA 20, if the MA 20 includes its MAID in the transmissions. The operation of the data management system in FIG. 5 will be further explained and exemplified below with reference to FIGS 8A-8B.

FIG. 6 is a block diagram of an example UD 30. A control unit 31 ("control circuitry") is configured to implement logic for controlling the user device 30. In the illustrated example, the control unit 31 comprises a combination of a processor 31A and a memory 31B. The memory 31B may store program instructions for execution by the processor 31A for implementing the operation of the control unit 31. The program instructions may be supplied to the processor 31A on a computer-readable medium. The processor 31A may be a generic processor (CPU, etc.), a specialized processor (ASIC, etc.), or any combination thereof. The memory 31B may include volatile and/or non-volatile memory such as read only memory (ROM), random access memory (RAM) or flash memory.

The UD 30 further comprises a communication device 32 (denoted COMM in the following), which is at least partly configured in correspondence with COMM 22 of the MA 20 (FIG. 2). Thus, COMM 32 is configured to implement the same communication technique and protocol as COMM 22. In some embodiments, COMM 32 is operable in a first mode, in which it intercepts broadcasts, and a second mode, in which it establishes an end-to-end connection to the MA 20 and receives a stream of data on the end-to-end connection. COMM 32 may also be operable to communicate with a server, for example by wireless data transfer over a network, such as a WAN, LAN, PAN or any combination thereof. In the illustrated example, the UD 30 further comprises a feedback device 33 for providing one or more of audible, visible or tactile feedback to the user. Such a feedback device 33 may comprise one or more of a speaker, a buzzer, a vibrator, a display, a touch screen, an indicator lamp, etc. As understood from the foregoing, the feedback device 33 may be operated, by the control unit 31 to provide feedback representing RTD as received by COMM 32 from an MA 20. The UD 30 also comprises a power source 34 for supplying power to the electrical components of the UD 30. The power source 34 may include a battery, a fuel cell, etc.

The UD 30 may be a customized electronic device for use in the system of FIG. 5 or a generic device, such as a mobile phone, smartphone, tablet computer, etc. Such a generic device may be configured for use as a UD 30 in the system of FIG. 5 by a dedicated application program ("app") that is installed and executed by the control unit 31.

FIG. 7A is a flow chart of an example method 700 performed by an MA 20 in the system of FIG. 5. The method 700 will be described with reference to the MA 20 in FIG. 2 and assuming that the steps are performed by the control unit 21.

In step 701, the start of an exercise activity is detected by use of the IMD 23. For example, step 701 may involve detecting a characteristic change in the signal from the IMU 23 or in one or more parameters derived from the signal. For example, the characteristic change may correspond to a change in acceleration, velocity, orientation or position. In an alternative, step 701 involves receiving a wake-up (WU) signal from the IMD 23, which is thus configured to detect the characteristic change and transmit the WU signal.

After detecting the start of an exercise activity in step 701, step 702 is performed. In step 702, COMM 22 is operated to detect if a UD is present or absent at the exercise device 10 when the exercise activity is started. In one example, COMM 22 transmits a query signal for receipt by a nearby UD and listens for a reply signal transmitted by the UD in response to the query signal. If a reply signal is received, presence of a UD is inferred, otherwise absence of a UD is inferred. In another example, COMM 22 listens for a beacon signal that is transmitted (broadcast) by a UD. If a beacon signal is received, presence of a UD is inferred, otherwise absence of a UD is inferred. The UD 30 may, for example, be configured to start listening for the query signal or transmitting the beacon signal when the user has performed a docking operation. To save power, COMM 22 may be in a low-power state by default and switched into a communication state in step 702, when the start of an exercise activity has been detected in step 701.

Steps 703 and 704 are performed if presence of a UD is detected in step 702. In step 703, the AMD 24 is operated to generate measurement data, MD, that represents the exercise activity. As noted above with reference to FIG. 2, the AMD 24 is in the sleep mode by default. Thus, by step 703, the AMD 24 is selectively switched into the active mode, in which it generates and outputs MD. In step 704, COMM 22 is operated to transmit first exercise data, ED1, which represents MD, for receipt by the UD 30. In the context of FIG. 5, ED1 corresponds to the real-time data, RTD. Thus, in some embodiments, step 704 is performed repeatedly to generate and transmit ED1 to represent the on-going exercise activity so as to allow the UD to provide real-time feedback to the user of the exercise device. In some embodiments, ED1 is streamed over an end-to-end connection to the UD, for example in the form of a time sequence of momentary positions, to represent a movement trajectory of the exercise device or a moveable element in the exercise device. Thereby, the UD is enabled to operate its feedback device 33 (FIG. 6) to present the movement trajectory in real-time to the user. In some embodiments, ED1 is broadcast for receipt by the UD, for example in the form of an indication that an exercise movement ("repetition") has been started or completed. Thereby, the UD is enabled to operate its feedback device 33 (FIG. 6) to present in real-time the current number of repetitions performed by the user within a set during the exercise activity. Alternatively or additionally, if the repetitions are counted by the MA 20, ED1 may be repeatedly transmitted to include a current count of repetitions. It is also conceivable, whether ED1 is streamed or broadcast, that ED1 designates the load that is lifted during the repetitions.

Step 705 is performed if absence of a UD is determined in step 702. The absence may be the result of an erroneous start detection in step 701, for example caused by vibrations being transferred to the exercise device from the surroundings or an unintentional displacement of the exercise device by a user. Another cause may be that an exercise activity is started by a user that does not have a UD configured for use in the data management system. In step 705, second exercise data, ED2, that represents the exercise activity is calculated by use of a measurement signal, S 1, from the IMD. In some embodiments, the AMD is in its default sleep mode and is thus disabled during step 705. This means that ED2 is generated in a power-efficient way, based only on S1 from the IMD. In other embodiments, the AMD is in the intermediate mode during step 705. This allows the calculation of ED2 in step 705 to be based on both S 1 from IMD and MD from the AMD in the intermediate mode. Such fusion of sensor data from the IMD and the AMD may increase the accuracy of ED2, for example if the performance of the IMD is limited. Here, it is assumed that ED2 cannot be calculated based solely on MD as generated by the AMD in the intermediate mode, but can be calculated when such MD is combined with S 1.

In the context of FIG. 5, ED2 corresponds to the summary usage data, SUD. Thus, ED2 contains a summary of the exercise activity, for example a count of the total number of repetitions performed during the exercise activity. The skilled person understands that a repetition may be detected in different ways in the measurement signal S1 from the IMD, for example depending on exercise device. In the example of the exercise machine in FIGS 3A-3B, relative positions derived from accelerometer data may be tracked to detect when the selected weights 11a leave and return to the rest state, respectively, and/or based on velocity values derived from the accelerometer data.

In some embodiments, to save power, ED2 is stored in internal memory of the MA 20 (cf. 21B in FIG. 2), for later retrieval. In other embodiments, in accordance with FIG. 5 and represented by step 706 in FIG. 7A, COMM 22 is operated to transmit ED2 for receipt by the OD 40.

It is understood that the example method 700 will save considerable power in the MA 20 by avoiding unnecessary power consumption by the AMD 24. Still, the MA 20 is capable of determining and providing summary usage data (ED2), should the exercise device be used by someone that does not have a UD for use in the data management system. ED2 is determined by the MA 20 in a power-efficient way, by use of the IMD 23, which is anyway present in the MA 20 and used for detecting the start of exercise activities.

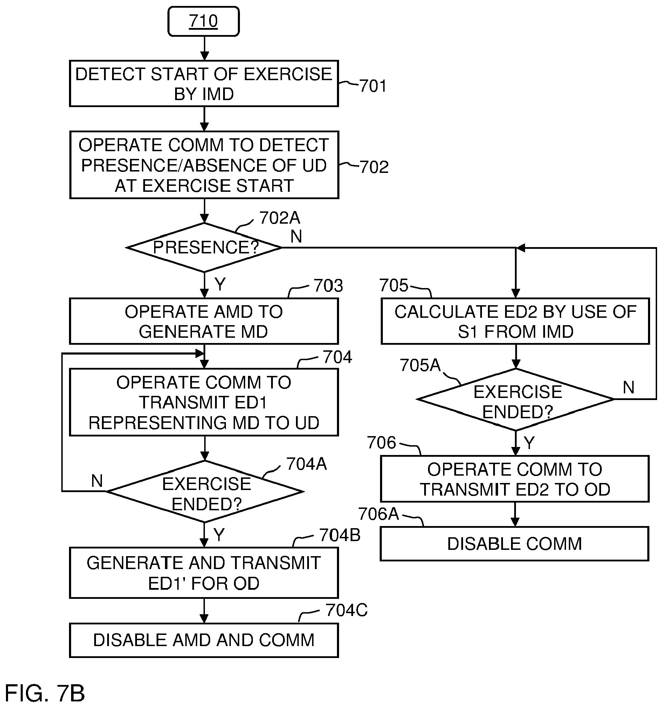

It may seem that the method 700 does not provide summary usage data for exercise activities performed in the presence of a UD. However, as noted with reference to FIG. 5, the OD 40 may autonomously determine such usage data based on intercepted transmissions of ED1 from the MA 20. This presumes that step 703 involves transmitting ED1 by broadcast, instead of or in addition to transmitting ED1 to the UD over an end-to-end connection. In an alternative, exemplified below with reference to FIG. 7B, the MA 20 may generate summary usage data (ED1') based on ED1 and transmit ED1' for receipt of the OD 40.

The method 710 in FIG. 7B is an expanded version of the method 700 in FIG. 7A. The method 710 includes that same steps 701-706 as the method 700. When start of exercise activity has been detected (step 701) and presence of a UD is detected (step 702), step 702A proceeds to operate the AMD in the active mode to generate MD (step 703) and to operate COMM 22 to generate and transmit ED1 for receipt by the UD (step 704). Here, it is assumed that the AMD, when activated in step 703, generates MD as a stream (time sequence) of values, for example position or distance values. In the illustrated example, step 704 is repeated to generate and transmit ED1 until termination of the exercise activity is detected in step 704A. In some embodiments, step 704A involves processing MD for detection of the termination. The skilled person understands that the termination may be detected in different ways depending on exercise device and AMD. For example, a termination may be detected if the exercise device is in a rest state for a predefined time period (cf. FIG. 3A). In an alternative, the user may signal the termination to the MA 20, for example by pressing a dedicated switch or button on the MA 20. In step 704B, which is performed when a termination is detected, COMM 22 is operated to transmit summary data, ED1', for receipt by the OD 40. In some embodiments, ED1' contains a summary of the exercise activity and is thus summary usage data, similar to ED2 as described above. After step 704B, the AMD is switched to the sleep mode in step 704C and is thus disabled. In step 704C, COMM 22 may also set in its low-power state.

If presence of a UD is not detected in step 702, step 702A proceeds to determine ED2 by use of S1 from the IMD, optionally in combination with MD from the AMD in the intermediate mode (step 705). Here, it is assumed that the IMD repeatedly generates S1, for example subsequent to step 701. In the illustrated example, step 705 is performed to determine ED2 until a termination of the exercise activity is detected in step 705A. In some embodiments, step 705A involves processing S1, and optionally MD from the AMD in intermediate mode, for detection of the termination. For example, a termination may be detected if one or more inertial parameters indicate that the exercise device 10 is in a rest state for a predefined time period (cf. FIG. 3A). In an alternative, the user may signal the termination to the MA 20, for example by pressing a dedicated switch or button on the MA 20. When a termination is detected, COMM 22 is operated to transmit ED2 for receipt by the OD 40 (step 706). Further, if the AMD is in the intermediate mode, the AMD is switched into sleep mode. After step 706, COMM 22 may be set in its low-power state in step 706A.

FIG. 8A is a sequence diagram of example operations performed in the system of FIG. 5 when a UD 30 is present at the exercise device 10. In the illustrated example, the MA 20 is represented by an IMD 23, an AMD 24 and a chip 21' that includes the control unit 21 and COMM 22 (cf. FIG. 2). In step 800, a docking operation is performed to associate the UD with the exercise device, for example as described hereinabove. In step 801, the IMD operates to detect start of an exercise activity. In step 802, the IMD detects such a start and outputs a wake-up (WU) signal. In step 803, the chip 21' receives the WU signal and thereby detect the start. Step 803 corresponds to step 701. In step 804, the chip 21' leaves the low-power state, thereby causing COMM 22 to be in the communication state. In step 805, the chip 21' broadcasts a connection request (query signal), RQ. In step 806, the UD receives RQ. In step 807, the UD generates a response message (reply signal), RP. In some embodiments, the UD will only generate RP if an identifier (MAID) in RQ matches an identifier that the UD received from a server as part of the docking operation. In step 808, the UD broadcasts RP. In step 809, the chip 21' receives RP. Steps 805, 809 correspond to step 702 and result in a detection of UD presence. In step 810, provided that RP is received, the chip 21' transmits a power-up (PU) signal to the AMD, causing the AMD to switch from sleep mode to active mode in step 811. Step 810 corresponds to step 703. In step 812, the chip 21' and the UD establish an end-to-end connection. In some embodiments, RQ includes a request for establishment of an end-to-end connection with the UD. In some embodiments, RP comprises an acceptance to establish the end-to-end-connection. In step 813, the AMD provides MD to the chip 21', which processes MD for generation of ED1 in step 814. In step 815, the chip 21' transmits the resulting ED1 on the end-to-end connection. Steps 813-815 correspond to step 704. The UD receives ED1 in step 816 and operates its feedback device to present ED1 (or data derived therefrom) in step 817. As shown, steps 813-817 are repeated during the course of the exercise activity. Each ED1 is generated based on the latest MD. Thus, ED1 will differ between different executions of step 815. In accordance with examples given above, each ED1 may include one or more momentary positions/distances, an indication of a start or completion of a repetition, a count of the number of repetitions performed so far within a set, etc. In step 818, the chip 21' and the UD detect that the exercise activity is terminated. The chip 21' may detect the termination as described above with reference to step 704A. The UD may detect the termination by processing of ED1 or be informed of the termination by the chip 21'. The chip 21' and the UD then terminates the end-to-end connection. In step 819, which corresponds to step 704C, the chip 21' transmits a power-down (PD) signal to the AMD, causing the AMD to switch from active mode to sleep mode in step 820. In step 821, which corresponds to step 704B, the chip 21' generates and broadcasts ED1'. In step 822, OD 40 receives ED1'. In step 823, OD 40 forwards ED 1', or part thereof, to the back-end device (not shown). In step 824, the chip 21' enters its low-power state (cf. step 704C).

As noted above, step 821 may be omitted. The OD may instead listen for ED1 as transmitted by the chip 21' in step 815 and then compile all the received instances of ED1 into ED 1'.

As also noted above, step 812 may be omitted. The chip 21' may instead broadcast ED1 in each execution of step 815. In a further alternative, the chip 21' transmits ED1 in step 815 both by broadcast and over the end-to-end connection.

FIG. 8B is a sequence diagram of example operations performed in the system of FIG. 5 when a UD 30 is not present at the exercise device 10. Steps 801-805 are performed as described for FIG. 8A and will not be repeated. In the illustrated example, RQ is received by a UD which is not configured for use in the data management system. Thus, although RQ is received in step 806 and parsed in step 807, the UD will decide not to send an RP. In another example, the UD will not generate RP if an identifier (MAID) in RQ does not match an identifier that the UD received from a server as part of the docking operation (cf. step 800 in FIG. 8A). In yet another example, no UD is present to receive RQ. Meanwhile, by step 830, the IMD is providing a measurement signal S1 to the chip 21'. Although not shown in FIG. 8B, the chip 21' may transmit a power-up signal to the AMD, causing the AMD to switch from sleep mode into intermediate mode, if no RQ is received. The chip 21' receives (step 831) and operates (step 832) on S 1, and optionally MD from the AMD in intermediate mode, for generation of summary usage data, ED2. In one example, S1 is processed, optionally together with MD (if present), for detection of repetitions during the exercise activity, for example as described with reference to step 705. In step 833, the chip 21' detects that the exercise activity is terminated. The chip 21' may detect the termination as described with reference to step 705A. In step 834, which corresponds to step 706, the chip 21' broadcasts ED2. In step 835, the OD receives ED2. In step 836, the OD forwards ED2, or part thereof, to the back-end device (not shown). In step 837, the chip 21' enters its low-power state (cf. step 706A), optionally after setting the AMD in the sleep mode.

While the subject of the present disclosure has been described in connection with what is presently considered to be the most practical embodiments, it is to be understood that the subject of the present disclosure is not to be limited to the disclosed embodiments, but on the contrary, is intended to cover various modifications and equivalent arrangements included within the spirit and the scope of the appended claims.

Further, while operations are depicted in the drawings in a particular order, this should not be understood as requiring that such operations be performed in the particular order shown or in sequential order, or that all illustrated operations be performed, to achieve desirable results.

In the following, clauses are recited to summarize some aspects and embodiments as disclosed in the foregoing.

C1. A monitoring arrangement for use with an exercise device (10), said monitoring arrangement comprising: a communication device (22) for wireless communication; an inertial measurement device (23), which is responsive to an exercise activity of the exercise device (10); and an activity measurement device (24), which is disabled by default and is operable to quantify the exercise activity; wherein the monitoring arrangement is configured to: detect, by use of the inertial measurement device (23), a start of the exercise activity; operate, upon detection of the start of the exercise activity, the communication device (22) to determine a presence or an absence of a mobile device (30) at the exercise device (10) at the start of the exercise activity; operate, upon determining the presence of the mobile device (30) at the start of the exercise activity, the activity measurement device (24) in an active mode to generate measurement data (MD) that represents the exercise activity, and operate the communication device (22) to transmit first exercise data (ED1) that represents the measurement data (MD) for receipt by the mobile device (30); and calculate, upon determining the absence of the mobile device (30) at the start of the exercise activity, second exercise data (ED2) that represents the exercise activity by use of a measurement signal (S 1) from the inertial measurement device (23) while the activity measurement device (24) is disabled or in an intermediate mode with lower power consumption than the active mode.

C2. The monitoring arrangement of C1, which is further configured to, upon determining the absence of the mobile device (30), operate the communication device (22) to transmit the second exercise data (ED2) for receipt by one or more receiving devices (40) installed in a facility that houses the exercise device (10).

C3. The monitoring arrangement of C2, which is configured to transmit the second exercise data (ED2) upon detecting a termination of the exercise activity, wherein the monitoring arrangement is configured to detect the termination by processing the measurement signal (S1) from the inertial measurement device (23), optionally in combination with measurement data (MD) generated by the activity measurement device in the intermediate mode.

C4. The monitoring arrangement of C3, which is configured to calculate the second exercise data (ED2) based on the measurement signal (S1) from the inertial measurement device (23) and the measurement data (MD) from the activity measurement device (32) in the intermediate mode.

C5. The monitoring arrangement of any one of C2-C4, which is further configured to, upon determining the presence of the mobile device (30), process the measurement data (MD) from the activity measurement device (24) in the active mode for detection of a termination of the exercise activity, and operate, upon detection of the termination, the communication device (22) to transmit summary data (ED1') representative of the first exercise data (ED1) for receipt by the one or more receiving devices (40).

C6. The monitoring arrangement of any preceding clause, wherein the activity measurement device (24) is operable to determine a momentary position of the exercise device (10) or a momentary position of one or more weights (11) in the exercise device (10).

C7. The monitoring arrangement of C6, wherein the first exercise data (ED1) represents a movement trajectory of the exercise device (10), or the one or more weights (11) in the exercise device (10), during the exercise activity.

C8. The monitoring arrangement of C6 or C7, wherein the first exercise data (ED1) comprises at least one of: an indication of a start or completion of an exercise movement performed as part of the exercise activity, a count of repetitive exercise movements performed during the exercise activity, or a load lifted during an exercise movement.

C9. The monitoring arrangement of any preceding clause, which is configured to operate the communication device to transmit the first exercise data (ED1) in time synchronization with the exercise activity.

C10. The monitoring arrangement of any preceding clause, wherein the second exercise data (ED2) is indicative of a count of exercise movements performed during the exercise activity.

C11. The monitoring arrangement of any preceding clause, which is configured to, upon detection of the start of the exercise activity, operate the communication device (22) to broadcast a connection request (RQ), detect the presence of the mobile device (30) when a response message (RP) from the mobile device (30) is received by the communication device (22), and otherwise detect the absence of the mobile device (30).

C12. The monitoring arrangement of C11, wherein the connection request (RQ) comprises a request for establishment of an end-to-end connection with the mobile device (30).

C13. The monitoring arrangement of C12, which is configured to, upon receipt of the response message (RP), establish the end-to-end connection with the mobile device (30), and transmit the first exercise data (ED1) in real-time to the mobile device (30) on the end-to-end connection.

C14. The monitoring arrangement of any preceding clause, which is configured to operate the communication device (22) to transmit the first exercise data (ED1) by broadcast during the exercise activity.

C15. The monitoring arrangement of any preceding clause, wherein the communication device (22) has a default low-power state and a communication state, and wherein the monitoring arrangement is configured to set the communication device (22) in the communication state upon detection of the presence of the mobile device (30) at the start of the exercise activity.

C16. The monitoring arrangement of any preceding clause, wherein the communication device (22) is configured for wireless short-range communication.

C17. The monitoring arrangement of any preceding clause, wherein the activity measurement device (24) is configured to measure a momentary vertical position of at least one of a plurality of stacked weights (11) in the exercise device (10).

C18. The monitoring arrangement of C17, wherein the activity measurement device (24) comprises a range sensor, which is arranged to measure a parameter representative of a distance (D) between said at least one of the plurality of stacked weights (11) and stationary element (24').

C19. An exercise device configured to be used in an exercise activity, said exercise device comprising the monitoring arrangement of any preceding clause.

C20. A computer-implemented method of operating a monitoring arrangement, said monitoring arrangement comprising a communication device for wireless communication; an inertial measurement device, which is responsive to an exercise activity of an exercise device; and an activity measurement device, which is disabled by default and is operable to quantify the exercise activity, said method comprising: detecting (701), by use of the inertial measurement device, a start of the exercise activity; operating (702), upon detecting the start of the exercise activity, the communication device to determine a presence or an absence of a mobile device at the exercise device at the start of the exercise activity; operating (703), upon determining the presence of the mobile device at the start of the exercise activity, the activity measurement device in an active mode to generate first exercise data that represents the exercise activity, and operating (704) the communication device to transmit the first exercise data for receipt by the mobile device; and calculating (705), upon determining the absence of the mobile device at the start of the exercise activity, second exercise data that represents the exercise activity by use of a measurement signal from the inertial measurement device while the activity measurement device is disabled or in an intermediate mode with lower power consumption than the active mode.

C21. A computer-readable medium comprising computer instructions which, when executed by processor circuitry (21A), cause the processor circuitry (21A) to perform the method of C20.

C22. A monitoring arrangement for use with an exercise device (10), the monitoring arrangement comprising: an inertial measurement device (23) configured to detect an exercise activity of the exercise device (10); an activity measurement device (24) separate from the inertial measurement device (23), the activity measurement device (24) having an active mode and a disabled mode, the activity measurement device (24) being operable to quantify the exercise activity of the exercise device (10) when in the active mode, and the activity measurement device (24) using less power when in the disabled mode than in the active mode; and a control unit (21) configured to: detect, via the inertial measurement device (23), a start of the exercise activity; cause the activity measurement device (24) to operate in the active mode upon detecting the start of the exercise activity; and operate the activity measurement device (24) to generate measurement data that represents the exercise activity when in the active mode; wherein the activity measurement device (24) is defaulted to the disabled mode to save power.

C23. The monitoring arrangement according to C22, further comprising a communication device (22), wherein the control unit (21) is further configured to transmit first exercise data that represents the measurement data from the activity measurement device (24) via the communication device (22).

C24. The monitoring arrangement according to C23, wherein the communication device (22) is configured to communicate wirelessly.

C25. The monitoring arrangement according to C23 or C24, wherein the control unit (21) is further configured to determine a presence or absence of a mobile device (30) and, when the mobile device (30) is present, to transmit the first exercise data to the mobile device (30).

C26. The monitoring arrangement according to any one of C23-C25, wherein the control unit (21) is further configured to determine a presence or absence of a mobile device (30) and, when the mobile device (30) is absent, to calculate second exercise data that represents the exercise activity using a measurement signal from the inertial measurement device (23), the second exercise data being different than the first exercise data.

C27. The monitoring arrangement according to C26, wherein the first exercise data is real-time data and the second exercise data is non-real-time data.

C28. The monitoring arrangement according to C26 or C27, wherein the control unit (21) is further configured to transmit the second exercise data to a receiver device (40) via the communication device (22), the receiver device (40) being different from the mobile device (30).

C29. The monitoring arrangement according to any one of C26-C28, wherein the control unit (21) is further configured to detect a termination of the exercise activity and to transmit the second exercise data when the termination of the exercise activity is detected.

C30. The monitoring arrangement according to C29, wherein the termination is detected using the measurement signal from the inertial measurement device (23).

C31. The monitoring arrangement according to any one of C26-C28, wherein the control unit (21) causes the activity measurement device (24) to operate in an intermediate mode when the mobile device (30) is absent, the activity measurement device (24) using less power when in the intermediate mode than in the active mode and more power when in the intermediate mode than in the disabled mode.

C32. The monitoring arrangement according to C31, wherein the control unit (21) is further configured to detect a termination of the exercise activity based at least in part on the measurement data from the activity measurement device (24) when operated in the intermediate mode, and to transmit the second exercise data when the termination of the exercise activity is detected.

C33. The monitoring arrangement according to C31 or C32, wherein the control unit (21) is further configured to calculate second exercise data that represents the exercise activity also using the measurement data from the activity measurement device (24) when operated in the intermediate mode.

C34. The monitoring arrangement according to any one of C26-C33, wherein the second exercise data is indicative of a count of exercise movements performed during the exercise activity.

C35. The monitoring arrangement according to any one of C23-C34, wherein the first exercise data that represents the measurement data from the activity measurement device (24) is transmitted in time synchronization with the exercise activity.

C36. The monitoring arrangement according to any one of C22-C35, wherein the activity measurement device (24) is operable to determine a momentary position of one of the exercise device (10) and a weight of the exercise device (10).

C37. The monitoring arrangement according to any one of C23-C36, wherein the first exercise data represents a movement trajectory of the one of the exercise device (10) and the weight of the exercise device (10).

C38. The monitoring arrangement according to C37, wherein the first exercise data comprises at least one of: an indication of a start of an exercise movement performed as part of the exercise activity, an indication of a completion of the exercise movement performed as part of the exercise activity, a count of repetitive exercise movements performed during the exercise activity, and an indication of a load being lifted during an exercise movement.

C39. A method for monitoring exercise activity for an exercise device (10), the method comprising: detecting the exercise activity via an inertial measurement device (23); detecting, via a control unit (21), a start of the exercise activity from the inertial measurement device (23); causing an activity measurement device (24) to operate in an active mode when the start of exercise activity is detected, the activity measurement device (24) being defaulted to a disabled mode that uses less power than the active mode when the start of exercise activity is undetected; generating, via the activity measurement device (24) when in the active mode, measurement data that quantifies the exercise activity; and communicating a first exercise data that represents the measurement data from the activity measurement device (24) when the activity measurement device (24) is in the active mode.

C40. The method according to C39, further comprising determining whether a mobile device (30) separate from the exercise device (10) is present or absent and, when the mobile device (30) is absent, calculating and communicating a second exercise data that represents the exercise activity using a measurement signal from the inertial measurement device (23), the second exercise data being different than the first exercise data.

C41. The method according to C40, wherein the first exercise data is real-time data and the second exercise data is non-real-time data, and wherein the second exercise data is communicated to a receiver device (40) that is different from the mobile device (30).

The functional block diagrams, operational sequences, and flow diagrams provided in the Figures are representative of exemplary architectures, environments, and methodologies for performing novel aspects of the disclosure. While, for purposes of simplicity of explanation, the methodologies included herein may be in the form of a functional diagram, operational sequence, or flow diagram, and may be described as a series of acts, it is to be understood and appreciated that the methodologies are not limited by the order of acts, as some acts may, in accordance therewith, occur in a different order and/or concurrently with other acts from that shown and described herein. For example, those skilled in the art will understand and appreciate that a methodology can alternatively be represented as a series of interrelated states or events, such as in a state diagram. Moreover, not all acts illustrated in a methodology may be required for a novel implementation.

This written description uses examples to disclose the invention, including the best mode, and also to enable any person skilled in the art to make and use the invention. Certain terms have been used for brevity, clarity, and understanding. No unnecessary limitations are to be inferred therefrom beyond the requirement of the prior art because such terms are used for descriptive purposes only and are intended to be broadly construed. The patentable scope of the invention is defined by the claims and may include other examples that occur to those skilled in the art. Such other examples are intended to be within the scope of the claims if they have features or structural elements that do not differ from the literal language of the claims, or if they include equivalent features or structural elements with insubstantial differences from the literal languages of the claims.

## Claims

1. A monitoring arrangement for use with an exercise device (10), the monitoring arrangement comprising:
an inertial measurement device (23) configured to detect an exercise activity of the exercise device (10);
an activity measurement device (24) separate from the inertial measurement device (23), the activity measurement device (24) having an active mode and a disabled mode, the activity measurement device (24) being operable to quantify the exercise activity of the exercise device (10) when in the active mode, and the activity measurement device (24) using less power when in the disabled mode than in the active mode; and
a control unit (21) configured to:
detect, via the inertial measurement device (23), a start of the exercise activity;
cause the activity measurement device (24) to operate in the active mode upon detecting the start of the exercise activity; and
operate the activity measurement device (24) to generate measurement data that represents the exercise activity when in the active mode;
wherein the activity measurement device (24) is defaulted to the disabled mode to save power.

2. The monitoring arrangement according to claim 1, further comprising a communication device (22), wherein the control unit (21) is further configured to transmit first exercise data that represents the measurement data from the activity measurement device (24) via the communication device (22).

3. The monitoring arrangement according to claim 2, wherein the communication device (22) is configured to communicate wirelessly.

4. The monitoring arrangement according to claim 2 or 3, wherein the control unit (21) is further configured to determine a presence or absence of a mobile device (30) and, when the mobile device (30) is present, to transmit the first exercise data to the mobile device (30).

5. The monitoring arrangement according to any one of claims 2-4, wherein the control unit (21) is further configured to determine a presence or absence of a mobile device (30) and, when the mobile device (30) is absent, to calculate second exercise data that represents the exercise activity using a measurement signal from the inertial measurement device (23), the second exercise data being different than the first exercise data.

6. The monitoring arrangement according to claim 5, wherein the first exercise data is real-time data and the second exercise data is non-real-time data.

7. The monitoring arrangement according to claim 5 or 6, wherein the control unit (21) is further configured to transmit the second exercise data to a receiver device (40) via the communication device (22), the receiver device (40) being different from the mobile device (30).

8. The monitoring arrangement according to any one of claims 5-7, wherein the control unit (21) is further configured to detect a termination of the exercise activity and to transmit the second exercise data when the termination of the exercise activity is detected.

9. The monitoring arrangement according to any one of claims 5-8, wherein the control unit (21) causes the activity measurement device (24) to operate in an intermediate mode when the mobile device (30) is absent, the activity measurement device (24) using less power when in the intermediate mode than in the active mode and more power when in the intermediate mode than in the disabled mode.

10. The monitoring arrangement according to claim 9, wherein the control unit (21) is further configured to calculate the second exercise data that represents the exercise activity also using the measurement data from the activity measurement device (24) when operated in the intermediate mode.

11. The monitoring arrangement according to any one of claims 5-10, wherein the second exercise data is indicative of a count of exercise movements performed during the exercise activity.

12. The monitoring arrangement according to any one of claims 2-11, wherein the first exercise data that represents the measurement data from the activity measurement device (24) is transmitted in time synchronization with the exercise activity.

13. The monitoring arrangement according to any preceding claim, wherein the activity measurement device (24) is operable to determine a momentary position of one of the exercise device (10) and a weight of the exercise device (10).

14. The monitoring arrangement according to any one of claims 2-13, wherein the first exercise data represents a movement trajectory of the one of the exercise device (10) and the weight of the exercise device (10), and wherein the first exercise data comprises at least one of: an indication of a start of an exercise movement performed as part of the exercise activity, an indication of a completion of the exercise movement performed as part of the exercise activity, a count of repetitive exercise movements performed during the exercise activity, and an indication of a load being lifted during an exercise movement.

15. A method for monitoring exercise activity for an exercise device (10), the method comprising:
detecting the exercise activity via an inertial measurement device (23);
detecting, via a control unit (21), a start of the exercise activity from the inertial measurement device (23);
causing an activity measurement device (24) to operate in an active mode when the start of exercise activity is detected, the activity measurement device (24) being defaulted to a disabled mode that uses less power than the active mode when the start of exercise activity is undetected;
generating, via the activity measurement device (24) when in the active mode, measurement data that quantifies the exercise activity; and
communicating a first exercise data that represents the measurement data from the activity measurement device (24) when the activity measurement device (24) is in the active mode.
